(19) Europäisches Patentamt
European Patent Office
Office européen des brevets

(11) **EP 3 708 080 A1**

(12) **EUROPEAN PATENT APPLICATION**

(43) Date of publication:
**16.09.2020 Bulletin 2020/38**

(21) Application number: **20150258.0**

(22) Date of filing: **03.01.2020**

(51) Int Cl.:
*A61B 5/145* (2006.01)     *A61B 5/00* (2006.01)
*G16H 50/20* (2018.01)     *G06N 3/02* (2006.01)
*A61B 5/1455* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.03.2019   US 201962819493 P
02.05.2019   US 201916402204**

(71) Applicant: **Samsung Electronics Co., Ltd.
Gyeonggi-do 16677 (KR)**

(72) Inventors:
• **Sakhaee, Elham**
  **Milpitas, CA California 95035 (US)**
• **Georgiadis, Georgios**
  **Porter Ranch, CA California 91326 (US)**
• **Liu, Liu**
  **Milpitas, CA California 95035 (US)**
• **Deng, Weiran**
  **Woodland Hills, CA California 91364 (US)**

(74) Representative: **Kuhnen & Wacker
Patent- und Rechtsanwaltsbüro PartG mbB
Prinz-Ludwig-Straße 40A
85354 Freising (DE)**

(54) **NEAR-INFRARED SPECTROSCOPY (NIR) BASED GLUCOSE PREDICTION USING DEEP LEARNING**

(57)     Provided is a recurrent neural network that predicts blood glucose level. The RNN includes a first long short-term memory (LSTM) network and a second LSTM network. The first LSTM network may include an input to receive near-infrared (NIR) radiation data and includes an output. The second LSTM network may include an input to receive the output of the first LSTM network and an output to output blood glucose level data based on the NIR radiation data input to the first LSTM network.

**Fig. 1**

**EP 3 708 080 A1**

**Description**

CROSS-REFERENCE TO RELATED APPLICATION

This application claims the priority benefit under of U.S. Provisional Application No. 62/819,493 filed on March 15, 2019, and U.S. Application No. 16/402,204 filed on May 2, 2019, the disclosure of which is incorporated herein by reference in its entirety.

TECHNICAL FIELD

The subject matter disclosed herein relates to neural networks. More specifically, the subject matter disclosed herein relates to a recurrent neural network that predicts blood glucose levels based on received near-infrared radiation data.

BACKGROUND

Regular monitoring and maintaining a blood glucose level may be crucial for preventing complications arising from diabetes. Most commercially available devices for blood glucose measurement are invasive or minimally invasive, and are inconvenient and may be painful. Detection of near-infrared (NIR) radiation may be used as an indicator of blood glucose level, and may be considered as a source of data for a non-invasive approach.

SUMMARY

An example embodiment provides a recurrent neural network to predict blood glucose level that may include a first long short-term memory (LSTM) and a second LSTM. The first long short-term memory (LSTM) network may include an input to receive near-infrared (NIR) radiation data and an output. The input NIR radiation data may include multichannel NIR radiation data. In one embodiment, the input NIR radiation data may include first overtone NIR radiation data. The second LSTM network may include input to receive the output of the first LSTM network and an output to output blood glucose level data based on the NIR radiation data input to the first LSTM network. In one embodiment, the recurrent neural network may include a denoiser filter coupled to the input of the first LSTM network that receives the NIR radiation data and outputs denoised NIR radiation data to the input of the first LSTM network. In one embodiment, the denoising filter may include a Savitzky-Golay filter.

An example embodiment provides a system to predict blood glucose level that may include an input interface, and a recurrent neural network. The input interface may receive multi-channel near-infrared (NIR) radiation data. In one embodiment, the input NIR radiation data may include first overtone NIR radiation data. The recurrent neural network may be coupled to the input interface, and may include a first long short-term memory (LSTM) and a second LSTM. The first long short-term memory (LSTM) network may include an input to receive near-infrared (NIR) radiation data and an output. The second LSTM network may include input to receive the output of the first LSTM network and an output to output blood glucose level data based on the NIR radiation data input to the first LSTM network. In one embodiment, the recurrent neural network may include a denoiser filter coupled to the input of the first LSTM network that receives the NIR radiation data and outputs denoised NIR radiation data to the input of the first LSTM network. In one embodiment, the denoising filter may include a Savitzky-Golay filter.

BRIEF DESCRIPTION OF THE DRAWING

In the following section, the aspects of the subject matter disclosed herein will be described with reference to exemplary embodiments illustrated in the figure, in which:

> FIG. 1 depicts a block diagram of a first example embodiment of a system to predict blood glucose level according to the subject matter disclosed herein;
> FIG. 2 is another block diagram of the first example embodiment of the system according to the subject matter disclosed herein;
> FIG. 3 depicts block diagram of an example embodiment of an LSTM network according to the subject matter disclosed herein; and
> FIG. 4 depicts a portion of the system depicted in FIG. 1 in which a data normalizer and a denoiser are part of the system.

DETAILED DESCRIPTION

In the following detailed description, numerous specific details are set forth in order to provide a thorough understanding of the disclosure. It will be understood, however, by those skilled in the art that the disclosed aspects may be practiced without these specific details. In other instances, well-known methods, procedures, components and circuits have not been described in detail not to obscure the subject matter disclosed herein.

Reference throughout this specification to "one embodiment" or "an embodiment" means that a particular feature, structure, or characteristic described in connection with the embodiment may be included in at least one embodiment disclosed herein. Thus, the appearances of the phrases "in one embodiment" or "in an embodiment" or "according to one embodiment" (or other phrases having similar import) in various places throughout this specification may not be necessarily all referring to the same embodiment. Furthermore, the particular features, structures or characteristics may be combined in any suitable manner in one or more embodiments. In this regard, as used herein, the word "exemplary" means "serving as an example, instance, or illustration." Any embodiment described herein as "exemplary" is not to be construed as necessarily preferred or advantageous over other embodiments. Additionally, the particular features, structures, or characteristics may be combined in any suitable manner in one or more embodiments. Also, depending on the context of discussion herein, a singular term may include the corresponding plural forms and a plural term may include the corresponding singular form. Similarly, a hyphenated term (e.g., "two-dimensional," "pre-determined," "pixel-specific," etc.) may be occasionally interchangeably used with a corresponding non-hyphenated version (e.g., "two dimensional," "predetermined," "pixel specific," etc.), and a capitalized entry (e.g., "Counter Clock," "Row Select," "PIXOUT," etc.) may be interchangeably used with a corresponding non-capitalized version (e.g., "counter clock," "row select," "pixout," etc.). Such occasional interchangeable uses shall not be considered inconsistent with each other.

Also, depending on the context of discussion herein, a singular term may include the corresponding plural forms and a plural term may include the corresponding singular form. It is further noted that various figures (including component diagrams) shown and discussed herein are for illustrative purpose only, and are not drawn to scale. Similarly, various waveforms and timing diagrams are shown for illustrative purpose only. For example, the dimensions of some of the elements may be exaggerated relative to other elements for clarity. Further, if considered appropriate, reference numerals have been repeated among the figures to indicate corresponding and/or analogous elements.

The terminology used herein is for the purpose of describing some example embodiments only and is not intended to be limiting of the claimed subject matter. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. The terms "first," "second," etc., as used herein, are used as labels for nouns that they precede, and do not imply any type of ordering (e.g., spatial, temporal, logical, etc.) unless explicitly defined as such. Furthermore, the same reference numerals may be used across two or more figures to refer to parts, components, blocks, circuits, units, or modules having the same or similar functionality. Such usage is, however, for simplicity of illustration and ease of discussion only; it does not imply that the construction or architectural details of such components or units are the same across all embodiments or such commonly-referenced parts/modules are the only way to implement some of the example embodiments disclosed herein.

It will be understood that when an element or layer is referred to as being on, "connected to" or "coupled to" another element or layer, it can be directly on, connected or coupled to the other element or layer or intervening elements or layers may be present. In contrast, when an element is referred to as being "directly on," "directly connected to" or "directly coupled to" another element or layer, there are no intervening elements or layers present. Like numerals refer to like elements throughout. As used herein, the term "and/or" includes any and all combinations of one or more of the associated listed items.

The terms "first," "second," etc., as used herein, are used as labels for nouns that they precede, and do not imply any type of ordering (e.g., spatial, temporal, logical, etc.) unless explicitly defined as such. Furthermore, the same reference numerals may be used across two or more figures to refer to parts, components, blocks, circuits, units, or modules having the same or similar functionality. Such usage is, however, for simplicity of illustration and ease of discussion only; it does not imply that the construction or architectural details of such components or units are the same across all embodiments or such commonly-referenced parts/modules are the only way to implement some of the example embodiments disclosed herein.

Unless otherwise defined, all terms (including technical and scientific terms) used herein have the same meaning as commonly understood by one of ordinary skill in the art to which this subject matter belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

As used herein, the term "module" refers to any combination of software, firmware and/or hardware configured to provide the functionality described herein in connection with a module. The software may be embodied as a software package, code and/or instruction set or instructions, and the term "hardware," as used in any implementation described herein, may include, for example, singly or in any combination, hardwired circuitry, programmable circuitry, state machine circuitry, and/or firmware that stores instructions executed by programmable circuitry. The modules may, collectively or individually, be embodied as circuitry that forms part of a larger system, for example, but not limited to, an integrated circuit (IC), system on-chip (SoC) and so forth. The various components and/or functional blocks disclosed herein may be embodied as modules that may include software, firmware and/or hardware that provide functionality described herein in connection with the various components and/or functional blocks.

Predicting blood glucose level based on NIR radiation data may be formulated as a regression problem in which NIR data (a vector time series) may be used as an input and blood glucose level (scalar time series) is a predicted output. The NIR data may be collected as multiband and/or multichannel data. At each time stamp, one NIR data input vector corresponds to a predicted output blood glucose level.

Training data (NIR data paired with a corresponding blood glucose level) may be collected for training a recurrent neural network. Typically, training data may be hard to collect and may be limited in quantity. Previously, a partial least squares (PLS) regression has been used to fit a neural network to training data. PLS may work well with clean data (no noise); however, PLS may perform poorly with real-world data that contains noise. Additionally, a PLS regression does not explicitly take into account information contained in data that was previously regressed, thereby making a trend-based prediction, such as a blood glucose level prediction, less reliable.

The subject matter disclosed herein provides a deep learning-based recurrent solution for a glucose-level prediction that uses a regressor network configuration that includes two stacked long short-term memory (LSTM) networks. In one embodiment, an autoencoder may be used at the input of the regression network for denoising purposes. In another embodiment, a denoising filter may be used in addition to or alternatively to an autoencoder. In another embodiment, if an autoencoder is coupled to the input of the denoiser filter, the autoencoder receives the NIR radiation data and outputs data codings corresponding to the NIR radiation data, wherein the denoiser filter receives the data codings corresponding to the NIR radiation data. Additionally, the training data may be augmented by interpolation of the training data to provide an enhanced set of training data that may be used with the regressor network configuration disclosed hereon.

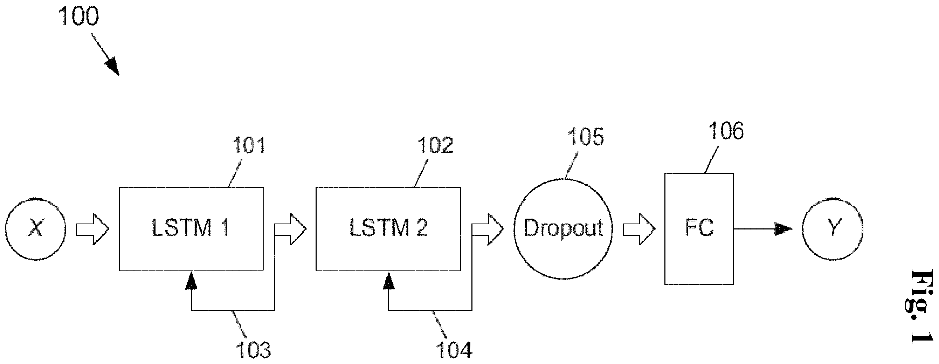

FIG. 1 depicts a block diagram of a first example embodiment of a system 100 to predict blood glucose level according to the subject matter disclosed herein. In one embodiment, the system 100 may be configured as a recurrent neural network that includes a first long short-term memory (LSTM) network 101 and a second LSTM network 102. The first LSTM network 101 receives NIR radiation data $X$, which may be multiband and/or multichannel NIR data. The output of the first LSTM network 101 is input to the second LSTM network 102. A portion of the cell state of the first LSTM network 101 is retained in the first LSTM network 101 at 103. Similarly, a portion of the cell state of the second LSTM network 102 is retained in the second LSTM network 102 at 104. The output of the second LSTM network 102 is input of a dropout 105. The dropout 105 may function as a regularizer. During training, the dropout 105 may set a random subset of the activations to zero to help the network generalize better. The output of the dropout 105 is input to a fully-connected layer FC 106 that may be used to perform regression of the glucose levels. The output $Y$ of the FC 106 is a single-dimensional prediction (i.e., a number) of a blood glucose level corresponding to the input NIR radiation data. In one embodiment, the dimensionality of the input NIR radiation data $X$ is 131, the dimensionality of the first layer is less than 41, and the dimensionality of the second layer is less than 20.

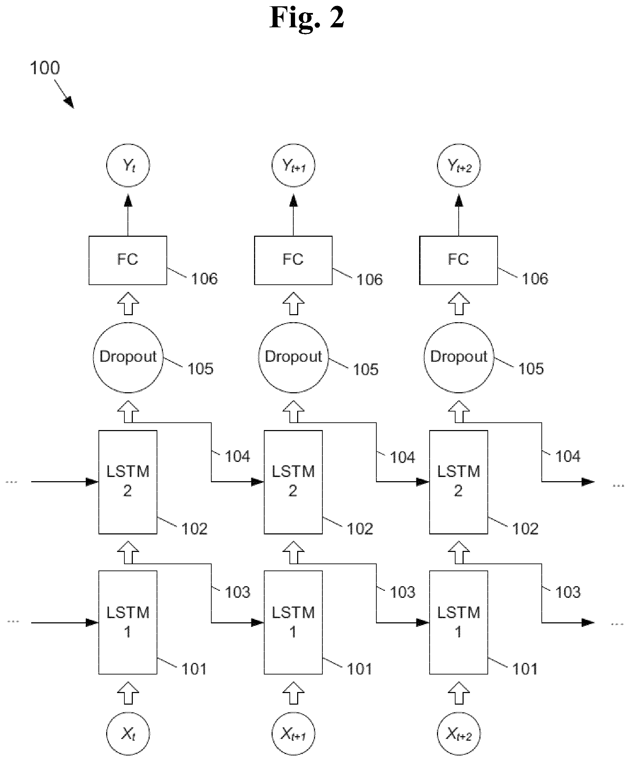

FIG. 2 is another block diagram of the first example embodiment of the system 100 according to the subject matter disclosed herein. FIG. 2 is the system 100 depicted as being unrolled in the time domain. The left-most system 100 shows the system 100 at time $t$. The input to the system 100 is a vector $X_t$ and the output is vector $Y_t$. The system 100 in the center of FIG. 2 shows the system 100 at time $t + 1$. The input to the system 100 is $X_{t+1}$ and the output is $Y_{t+1}$. The right-most system 100 of FIG. 2 shows the system 100 at time $t + 2$. The input to the system 100 is $X_{t+2}$ and the output is $Y_{t+2}$. At 103, a portion of the cell state of the first LSTM network 101 at time $t$ is retained in the first LSTM network 101 at time $t + 1$, and portion of the cell state of the first LSTM network 101 at time $t + 1$ is retained in the first LSTM network 101 at time $t + 2$. Similarly, at 104, a portion of the cell state of the second LSTM network 102 at time $t$ is retained in the second LSTM network 102 at time $t + 1$, and portion of the cell state of the second LSTM network 102 at time $t + 1$ is retained in the second LSTM network 102 at time $t + 2$.

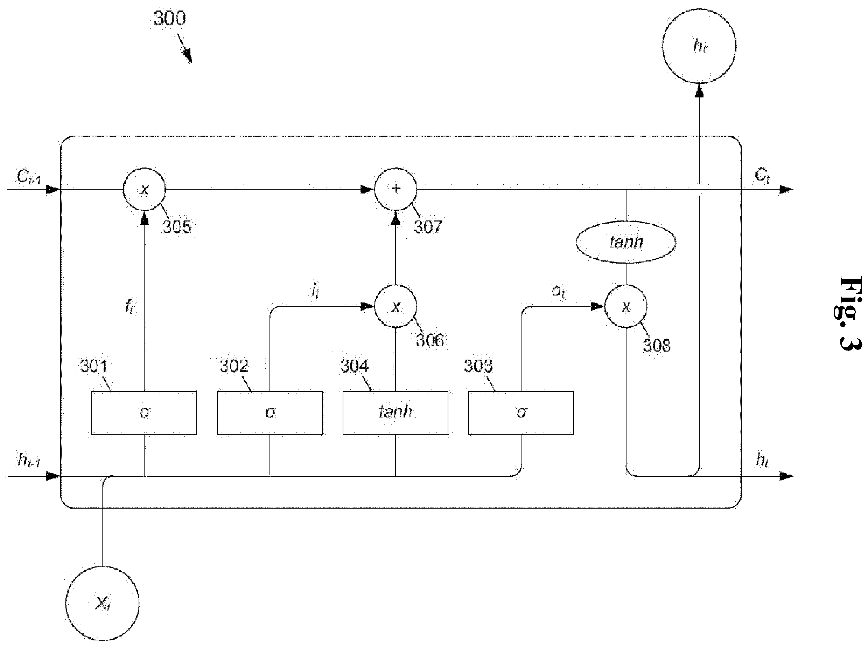

FIG. 3 depicts block diagram of an example embodiment of an LSTM network 300 according to the subject matter disclosed herein. The first and second LSTM networks 101 and 102 may be configured as the example embodiment of the LSTM network 300. The LSTM network 300 may include three gate layers 301-303 and a *tanh* layer 304.

The gate layer 301 functions as a forget gate layer to decide what information in the cell state $C$ that will be forgotten. The forget gate layer 301 may include a sigmoid layer that receives the previous output $h_{t-1}$ of the LSTM network 300 and a current input $X_t$. The forget gate layer 301 outputs $f_t$, which includes a value $f_t$ between 0 and 1 for each value in a previous cell state $C_{t-1}$, in which 0 represents a complete forget action of the value of the previous cell state and 1 represents a complete retention of the value of the previous cell state. The values of the output $f_t$ and the

previous cell state $C_{t-1}$ are multiplied by a multiplier 305. The output $f_t$ may be determined as

$$f_t = \sigma_g(W_f x_t + U_f h_{t-1} + b_f), \tag{1}$$

in which $\sigma_g$ is the sigmoid function of the input gate unit, $W_f$ is the size of the first hidden layer (i.e., first LSTM network 101) times the input feature size $d$, $U_f$ is the size of the first hidden layer squared, and $b_f$ is a constant associated with the forget gate unit. In one embodiment, the size of the first hidden layer may be 41.

The gate layer 302 functions as an input gate layer to decide what new information will be stored in the current cell state $C_t$. The input gate layer 302 may include a sigmoid layer that receives the previous output $h_{t-1}$ of the LSTM network 300 and the current input $X_t$, and outputs a vector $i_t$ as

$$i_t = \sigma_g(W_i x_t + U_i h_{t-1} + b_i), \tag{2}$$

in which $\sigma_g$ is the sigmoid function of the input gate unit, $W_i$ is the size of the first hidden layer (i.e., first LSTM network 101) times the input feature size $d = 131$, $U_f$ is the size of the first hidden layer squared, and $b_i$ is a bias value.

The layer 304 receives as an input the previous output $h_{t-1}$ of the LSTM network 300 and the current input $X_t$. The layer 304 outputs an intermediate cell state vector $c_t$ as

$$c_t = \sigma_c(W_c x_t + U_c h_{t-1} + b_c), \tag{3}$$

in which $\sigma_c$ is a sigmoid function, $W_c$ is a learnable parameter matrix of weight that having a size of the first hidden layer (i.e., first LSTM network 101) times the input feature size $d = 131$, $U_c$ is the size of the first hidden layer squared, and $b_C$ is a bias value constant.

The output $i_t$ of the input gate layer 302 and the intermediate cell state vector $c_t$ output from the layer 304 are multiplied by a multiplier 306. The output of the multiplier 305 and the output of the multiplier 306 are added by a summer 307 to form the current cell state $C_t$ of the LSTM network 300 as

$$C_t = f_t \cdot c_{t-1} + i_t \cdot \sigma_c(W_c x_t + U_c h_{t-1} + b_c) \tag{4}$$

The gate layer 303 functions as an output gate to determine what output $h_t$ will be output from the LSTM network 300. The output gate layer 303 receives as an input the previous output $h_{t-1}$ of the LSTM 300 and the current input $X_t$, and outputs $o_t$ as

$$o_t = \sigma_g(W_o x_t + U_o h_{t-1} + b_o), \tag{5}$$

in which $\sigma_g$ is the sigmoid function of the input gate unit, $W_o$ is the size of the first hidden layer (i.e., LSTM network 101) times the input feature size $d = 131$, $U_o$ is the size of the first hidden layer squared, and $b_o$ is a bias value constant.

A *tanh* function is applied to the cell state $C_t$. The output $o_t$ of the output gate layer 303 and the output of the *tanh* function are multiplied by a multiplier 308 to form the output $h_t$, which is an encoded representation that is similar to an output feature map of an activation function, as

$$h_t = o_t * tanh(C_t). \tag{6}$$

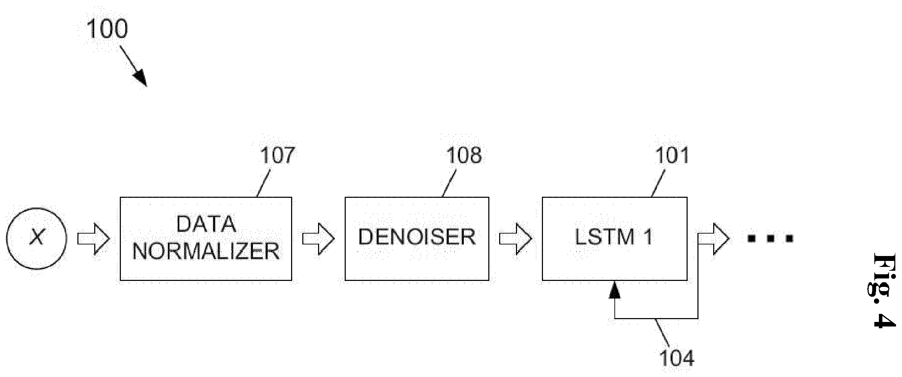

To train the system 100, training data may be used having NIR data paired with a corresponding blood glucose level. Preprocessing of the training data may be used to provide better quality data for fitting the model of system 100, which is purely data driven. Additionally, the raw NIR data may be denoised to prevent the system 100 from overfitting the noise. In one embodiment, a Savitzky-Golay filter may be used to smooth the NIR data. In yet another embodiment, normalization may be provided to provide better data distribution and provide an easier gradient descent for training purposes. A stochastic gradient descent (SGD) may be used during backpropagation to optimize an L2 loss function. FIG. 4 depicts a portion of the system 100 in which a data normalizer 107 and a denoiser 108 are part of the system 100. The training data may include 131 channels, and may include first overtone data. The output of the first LSTM network 101 is hi channels, and the output of the second LSTM network 102 is h2 channels.

In one embodiment, to further improve the performance and capability of generalization of model by increasing the training dataset size and variety, the input training data may be augmented by interpolation of the NIR data and blood glucose level pairs.

As will be recognized by those skilled in the art, the innovative concepts described herein can be modified and varied over a wide range of applications. Accordingly, the scope of claimed subject matter should not be limited to any of the specific exemplary teachings discussed above, but is instead defined by the following claims.

## Claims

1. A recurrent neural network to predict blood glucose level, the recurrent neural network comprising:

   a first long short-term memory (LSTM) network comprising an input to receive near-infrared (NIR) radiation data and an output; and
   a second LSTM network comprising an input to receive the output of the first LSTM network and an output to output blood glucose level data based on the NIR radiation data input to the first LSTM network.

2. The recurrent neural network of claim 1, further comprising a denoiser filter coupled to the input of the first LSTM network, the denoiser filter receiving the NIR radiation data and outputting denoised NIR radiation data to the input of the first LSTM network.

3. The recurrent neural network of claim 2, wherein the denoising filter comprises a Savitzky-Golay filter.

4. The recurrent neural network of claim 2, further comprising an autoencoder coupled to the input of the denoiser filter, the autoencoder receiving the NIR radiation data and outputting data codings corresponding to the NIR radiation data,
   wherein the denoiser filter receives the data codings corresponding to the NIR radiation data.

5. The recurrent neural network of claim 1, further comprising an autoencoder coupled to the input of the first LSTM network, the autoencoder receiving the NIR radiation data and outputting denoised NIR radiation data to the input of the first LSTM network.

6. The recurrent neural network of claim 1, wherein the input NIR radiation data comprises multichannel NIR radiation data.

7. The recurrent neural network of claim 6, wherein the input NIR radiation data comprises 131 channels.

8. The recurrent neural network of claim 1, wherein the input NIR radiation data comprises first overtone NIR radiation data.

9. A system to predict blood glucose level, the system comprising:

   an input interface to receive near-infrared (NIR) radiation data; and
   a recurrent neural network coupled to the input interface, the recurrent neural network comprising:

      a first long short-term memory (LSTM) network comprising an input to receive the NIR radiation data and an output; and
      a second LSTM network comprising an input to receive the output of the first LSTM network and an output to output blood glucose level data based on the NIR radiation data input to the first LSTM network.

10. The system of claim 9, further comprising a denoiser filter coupled to the input of the first LSTM network, the denoiser filter receiving the NIR radiation data and outputting denoised NIR radiation data to the input of the first LSTM network.

11. The system of claim 10, wherein the denoising filter comprises a Savitzky-Golay filter.

12. The system of claim 10, further comprising an autoencoder coupled to the input of the denoiser filter, the autoencoder receiving the NIR radiation data and outputting data codings corresponding to the NIR radiation data,

wherein the denoiser filter receives the data codings corresponding to the NIR radiation data.

13. The system of claim 9, further comprising an autoencoder coupled to the input of the first LSTM network, the autoencoder receiving the NIR radiation data and outputting denoised NIR radiation data to the input of the first LSTM network.

14. The system of claim 9, wherein the input NIR radiation data comprises multichannel NIR radiation data.

15. The system of claim 9, wherein the input NIR radiation data comprises first overtone NIR radiation data.

**Fig. 1**

# Fig. 2

# Fig. 3

EP 3 708 080 A1

100

**Fig. 4**

**EP 3 708 080 A1**

Europäisches Patentamt
European Patent Office
Office européen des brevets

# EUROPEAN SEARCH REPORT

Application Number

EP 20 15 0258

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2018/271448 A1 (BYNAM KIRAN [IN] ET AL) 27 September 2018 (2018-09-27) * paragraphs [0031], [0052] - [0055], [0076], [0086] * * figure 2 * ----- | 1-15 | INV. A61B5/145 A61B5/00 G16H50/20 G06N3/02 |
| Y | US 2018/196599 A1 (KIM JAEYOUNG [US] ET AL) 12 July 2018 (2018-07-12) * paragraphs [0003] - [0008] * ----- | 1-15 | ADD. A61B5/1455 |
| A | XINRUI LYU ET AL: "Improving Clinical Predictions through Unsupervised Time Series Representation Learning", ARXIV.ORG, CORNELL UNIVERSITY LIBRARY, 201 OLIN LIBRARY CORNELL UNIVERSITY ITHACA, NY 14853, 3 December 2018 (2018-12-03), XP080988102, * the whole document * ----- | 1-15 | |

| TECHNICAL FIELDS SEARCHED (IPC) |
|---|
| A61B G16H G06N |

The present search report has been drawn up for all claims

| Place of search | Date of completion of the search | Examiner |
|---|---|---|
| The Hague | 9 July 2020 | Faymann, Juan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

12

## ANNEX TO THE EUROPEAN SEARCH REPORT
## ON EUROPEAN PATENT APPLICATION NO.

EP 20 15 0258

This annex lists the patent family members relating to the patent documents cited in the above-mentioned European search report.
The members are as contained in the European Patent Office EDP file on
The European Patent Office is in no way liable for these particulars which are merely given for the purpose of information.

09-07-2020

| Patent document cited in search report | | Publication date | Patent family member(s) | | Publication date |
|---|---|---|---|---|---|
| US 2018271448 | A1 | 27-09-2018 | EP 3381368 A1 | | 03-10-2018 |
| | | | US 2018271448 A1 | | 27-09-2018 |
| US 2018196599 | A1 | 12-07-2018 | CN 108304914 A | | 20-07-2018 |
| | | | KR 20180083238 A | | 20-07-2018 |
| | | | US 2018196599 A1 | | 12-07-2018 |

EPO FORM P0459

For more details about this annex : see Official Journal of the European Patent Office, No. 12/82

**REFERENCES CITED IN THE DESCRIPTION**

**Patent documents cited in the description**

- US 62819493

- US 40220419